Europäisches Patentamt

European Patent Office

Office européen des brevets

(11) Numéro de publication: **0 407 291 A1**

# DEMANDE DE BREVET EUROPEEN

(21) Numéro de dépôt: 90401918.9

(22) Date de dépôt: 03.07.90

(51) Int. Cl.5: **C12Q 1/68, C12Q 1/48,**
//C12Q1/70,C07H21/04

(30) Priorité: 03.07.89 FR 8908873

(43) Date de publication de la demande:
09.01.91 Bulletin 91/02

(84) Etats contractants désignés:
AT BE CH DE DK ES FR GB GR IT LI LU NL SE

(71) Demandeur: **INSTITUT PASTEUR**
**25-28, rue du Docteur Roux**
**F-75724 Paris Cédex 15(FR)**

(72) Inventeur: **Griffais, Rémy**
**15 rue du Port Galand**
**F-92220 Bagneux(FR)**

(74) Mandataire: **Orès, Bernard et al**
**Cabinet ORES 6, Avenue de Messine**
**F-75008 Paris(FR)**

(54) **Perfectionnements apportés aux techniques d'amplification d'acide nucléique.**

(57) Perfectionnement apporté aux techniques d'amplification d'acides nucléiques, notamment par réaction de polymérisation en chaîne (PCR).

Ce procédé de détection et/ou d'identification d'une séquence d'acide nucléique ou d'un mélange de séquences d'acides nucléiques après mise en solution appropriée de l'échantillon biologique, pour extraire le/les acides nucléiques, comprend les étapes suivantes :

(1) une étape de destruction des extrémités 5' des séquences d'acide nucléique double brin présentes dans l'échantillon, par mise en contact dudit échantillon biologique avec un réactif approprié, actif à une température comprise entre 37°-42°C ;

(2) une étape d'amplification proprement dite par mise en contact de l'échantillon obtenu en (1) avec des réactifs appropriés, notamment des amorces d'amplification appropriées à l'amplification de la/les séquences cibles à détecter, en présence d'une ADN polymérase thermorésistante ;

(3) une étape de détection des séquences cibles spécifiques amplifiées.

EP 0 407 291 A1

# PERFECTIONNEMENTS APPORTES AUX TECHNIQUES D'AMPLIFICATION D'ACIDE NUCLEIQUE.

La présente invention est relative à un perfectionnement apporté aux techniques d'amplification d'acides nucléiques, notamment par réaction de polymérisation en chaîne (PCR).

L'une des premières méthodes d'amplification décrites, est la réaction de polymérisation en chaîne (PCR), développée par SAIKI R. et al. (Science, 1985, 230 , 1350). Cette technique permet notamment d'amplifier des séquences d'ADN simple brin ou double brin et est basée sur le fonctionnement cyclique d'une ADN polymérase, laquelle enzyme est capable de copier un brin d'ADN, utilisé comme matrice, en un brin complémentaire par élongation à partir de l'extrémité 3' OH libre d'une amorce oligonucléotidique.

La technique PCR consiste à effectuer n cycles successifs d'amplification, au cours desquels deux amorces dirigent l'amplification de la séquence d'ADN double brin qu'elles encadrent. Un cycle d'amplification est composé de trois étapes permettant de réaliser successivement la dénaturation de l'ADN (à une température comprise entre 90 et 95°C), l'hybridation des amorces (par exemple à une température comprise entre 37 et 75°C) et l'élongation des brins d'ADN par l'ADN polymérase.

Les bases technologiques de la PCR reposent sur deux points fondamentaux :
- l'utilisation de deux amorces oligonucléotidiques, l'une complémentaire du brin ADN+, l'autre du brin ADN- et dont les extrémités 3' sont en regard ;
- l'utilisation répétitive de l'activité d'une ADN polymérase appropriée.

La PCR permet alors l'utilisation, dans des conditions adéquates, de toutes les séquences néosynthétisées au cycle n comme matrice, pour l'ADN polymérase au cycle (n+1). Il en résulte une amplification exponentielle du nombre de séquences d'acides nucléiques cibles en fonction du nombre de cycles, conformément à la courbe d'amplification qui comprend une phase exponentielle où la quantité Q de séquences cibles obtenues peut être reliée à la quantité Qo de séquences cibles initiales, au facteur d'amplification x et au nombre de cycles n par la formule suivante : $Q = Qo (1 + x)^n$.

La PCR permet ainsi d'amplifier, de manière exponentielle, des séquences d'acides nucléiques, dites cibles, des milliers de fois.

Cette technique d'amplification est plus particulièrement décrite dans la Demande de Brevet européen CETUS 201 184, qui spécifie notamment que les deux amorces oligonucléotidiques mises en oeuvre dans la PCR sont de préférence simple brin et doivent être suffisamment longues pour amorcer la synthèse du produit d'extension en présence de l'ADN polymérase.

Un certain nombre d'améliorations ou de perfectionnements à cette technique ont été proposés et notamment, pour éviter l'addition d'enzymes à chaque cycle, une ADN polymérase pouvant résister à 100°C, la Taq polymérase, décrite dans la Demande de Brevet européen CETUS 258 017, est ajoutée dès le début du procédé et permet de réaliser un nombre important de cycles d'amplification consécutifs. La Taq polymérase a permis d'une part d'automatiser le procédé d'amplification et d'autre part d'augmenter la spécificité de l'amplification.

La PCR permet ainsi d'obtenir, sans clonage, une amplification considérable d'une séquence nucléique, dite séquence cible, donc un énorme gain en sensibilité. La séquence cible amplifiée est ensuite directement accessible à différents procédés d'analyse tels que le procédé de dot-blot, l'électrophorèse ou le séquençage. Deux Demandes de Brevet (Demandes de Brevet européen CETUS n° 200 362 et n° 229 701) décrivent plus particulièrement l'association amplification-hybridation pour la détection d'une séquence d'acide nucléique recherchée dans un échantillon.

Depuis, des variantes et/ou des perfectionnements ont été proposés et la PCR est actuellement largement utilisée tant en biologie moléculaire qu'en clinique (diagnostic de maladies génétiques et détection de microorganismes pathogènes). Cependant, l'utilisation en routine de cette technique a fait apparaître un inconvénient important, lié à son extrême sensibilité, à savoir l'émergence de faux-positifs, qui se sont révélés être liés, après une analyse soigneuse des résultats, à une contamination de l'échantillon à analyser.

Un certain nombre d'articles, parus depuis environ deux ans, décrivent de tels cas de contaminations et proposent des moyens pour les résoudre. Ces articles mettent en valeur un type de contamination inattendue, à savoir la contamination par des séquences identiques à celles recherchées.

- Y.M.D. LO et al., dans un article paru dans Lancet (1988, ii, 679), font apparaître cet inconvénient de la réaction de polymérisation en chaîne, dû à sa sensibilité et, pour pallier cet inconvénient important, préconisent de prendre un soin particulier dans la préparation de la matrice d'ADN avant l'amplification. LO et al. décrivent plus particulièrement un exemple de contamination dans un diagnostic de séquences HBV et montrent un résultat positif obtenu à partir d'échantillons négatifs : des séquences HBV ont été amplifiées à partir d'échantillons négatifs, une vérification de l'absence de séquences à détecter dans l'échantillon ayant été réalisée par un Southern Blot.

LO et al. ont analysé ce résultat comme étant une contamination des amorces utilisées, par des plasmides contenant l'insert HBV (plasmide pHBV130), dans la mesure où, lorsqu'ils se débarrassent des amorces contaminées, il n'y a plus d'amplifications faussement positives dans les échantillons négatifs.

LO et al. suggèrent que, dans la mesure où la PCR est de plus en plus utilisée dans le diagnostic, le problème des faux positifs, par contamination par des séquences identiques à celles à détecter, ne peut être négligé. LO et al. proposent de réaliser systématiquement un témoin dit négatif consistant uniquement en réactifs pour la PCR pour évaluer la contamination plasmidique éventuelle de ces réactifs.

LO et al. proposent également de vérifier l'existence d'une contamination plasmidique par amplification en présence d'amorces correspondant à des séquences placées de part et d'autre de la jonction vecteur-insert dudit plasmide. Cette procédure est spécialement nécessaire si des résultats conflictuels sont obtenus par les méthodes conventionnelles telles que le Southern Blot.

Il faut cependant noter que le problème de la contamination par la présence d'un plasmide vecteur du fragment cible ne se pose que dans le cadre des laboratoires de recherche alors que les laboratoires de diagnostic (laboratoires d'analyses médicales par exemple), effectuant des identifications en routine, disposent de réactifs prêts à l'emploi et qu'en ce cas, il est important d'éviter la contamination de la matrice par des produits autres que des plasmides (produits de la PCR, virus libres par exemple) pour la réalisation d'un diagnostic fiable, cette contamination ne pouvant être évitée, de l'avis de LO et al, à l'heure actuelle.

- R.A. GIBBS et al., dans un article paru dans Genes & Development, 1989, 3, 1095-1098, prodiguent les conseils suivants : isoler tous les réactifs utilisés pour la mise en oeuvre de la PCR des équipements utilisés pour analyser les produits obtenus ; utiliser des bouchons à vis et même aller jusqu'à congeler les contenus, avant d'effectuer la réaction finale, afin d'éviter la formation d'aérosols ; limiter le nombre de cycles d'amplification au minimum, à la fois pour réduire le risque d'amplifier de petites quantités de contaminants déjà présents et pour réduire la quantité totale de produits de réaction et disposer de jeux de pipettes différents pour chaque tâche.

- G. SARKAR et al., dans un article paru dans Nature, 1990, 343, 27, proposent, pour éviter cette contamination, de traiter l'échantillon aux rayons UV, avant d'ajouter la matrice d'ADN à amplifier.

- S. KWOK et al., dans un article paru dans Nature, 1989, 339, 237-238, proposent un certain nombre de "bonnes pratiques de laboratoire" pour contrôler et éviter la contamination : isoler physiquement les préparations et les produits pour PCR (pièces séparées, rayons UV...) ; autoclaver les tampons utilisés ; fractionner les réactifs en petits échantillons afin d'éviter un pipetage répété ; utiliser des gants de protection lors des manipulations ; éviter les projections ; utiliser des pipettes ne provoquant pas l'apparition d'aérosols contenant des échantillons d'ADN ; prémélanger les réactifs qui peuvent l'être afin de diminuer le nombre de manipulations contaminantes ; ajouter l'ADN en dernier ; choisir soigneusement les contrôles positifs et négatifs.

La contamination décrite par ces différents Auteurs est donc une contamination par des séquences identiques à celles à détecter, dites séquences cibles, issues, dans la majorité des cas, au laboratoire d'analyses médicales, d'amplifications antérieures, et qui génèrent un grand nombre de copies, entraînent une accumulation des fragments cibles amplifiés et aboutissent notamment à la présence ubiquitaire des séquences cibles dans le laboratoire d'analyses médicales. Cependant, l'ensemble des suggestions proposées par les différents Auteurs précités, pour éviter cette contamination, ont l'inconvénient d'être particulièrement contraignantes et difficiles à mettre en oeuvre en routine.

De plus, ces différentes suggestions ne permettent pas d'éliminer systématiquement la contamination due à la présence de séquences cibles issues d'amplifications antérieures.

C'est pourquoi, la Demanderesse s'est donné pour but de pourvoir à un procédé d'amplification de séquences cibles, qui répond mieux aux nécessités de la pratique que les procédés antérieurs, notamment en ce qu'il permet d'empêcher l'amplification exponentielle des acides nucléiques double brin contaminants, c'est-à-dire des séquences identiques aux séquences à détecter, amplifiées lors de précédents tests au laboratoire et qui pourraient entraîner des résultats faussement positifs.

On entend, au sens de la présente invention, par séquence contaminante, un fragment d'acide nucléique court qui correspond à un fragment d'ADN génomique à détecter (séquence cible) et qui possède à son extrémité 5' une amorce intégrée.

On entend, au sens de la présente invention, par séquence cible, une séquence définie par les amorces d'amplification qui l'encadrent, laquelle séquence est incluse dans un acide nucléique de séquence longue, ADN génomique notamment.

La présente invention a pour objet un procédé de détection et/ou d'identification d'une séquence d'acide nucléique ou d'un mélange de séquences d'acides nucléiques comprenant une amplification enzymatique, caractérisé en ce qu'après mise en solution appropriée de l'échantillon biologique, pour extraire le/les acides nucléiques, ledit procédé comprend les étapes suivantes :

(1) une étape de destruction des extrémités 5′ des séquences d'acide nucléique double brin présentes dans l'échantillon, par mise en contact dudit échantillon biologique avec un réactif approprié, actif à une température comprise entre 37° -42° C ;

(2) une étape d'amplification proprement dite par mise en contact de l'échantillon obtenu en (1) avec des réactifs appropriés, notamment des amorces d'amplification appropriées à l'amplification de la/les séquences cibles à détecter, en présence d'une ADN polymérase thermorésistante ;

(3) une étape de détection des séquences cibles spécifiques amplifiées.

Selon un mode de mise en oeuvre avantageux du procédé conforme à l'invention, le réactif de l'étape (1), dénommé réactif de discrimination entre séquences contaminantes et séquences cibles, est choisi dans le groupe qui comprend des produits chimiques convenables et des enzymes qui agissent en milieu alcalin faible.

Selon une disposition avantageuse de ce mode de mise en oeuvre, l'enzyme est une 5′ exonucléase appropriée, de préférence la 5′ lambda exonucléase.

Selon une modalité avantageuse de cette disposition, les amorces mises en oeuvre pour l'amplification de l'étape (2) sont phosphorylées à leurs extrémités 5′.

La lambda exonucléase a la propriété de détruire les extrémités 5′ des acides nucléiques double brin lorsque lesdites extrémités sont phosphorylées.

L'action de cette 5′ lambda exonucléase est plus particulièrement décrite dans les articles de J. W. LITTLE (J. Biol. Chem., 1967, 242 , 4, 672-686).

La mise en oeuvre de l'étape 1 précitée est cruciale pour éviter les contaminations par des séquences identiques à celles à détecter.

En effet, comme précisé ci-dessus, les séquences contaminantes correspondent à des séquences relativement courtes, comportant à leurs extrémités, les amorces d'amplification utilisées lors d'amplifications précédentes (produits d'extension d'amorce), alors que les séquences cibles à détecter, définies par les amorces d'amplification qui les encadrent (même amorces que celles incorporées dans les séquences contaminantes), sont incluses dans un acide nucléique de séquence longue (ADN génomique notamment).

Au cours de l'étape (1) du procédé conforme à l'invention, le réactif de discrimination et plus particulièrement la 5′ exonucléase, qui a la propriété de détruire les nucléotides d'un acide nucléique double brin uniquement, de proche en proche, dans le sens 5′→3′, conduit à l'obtention d'acides nucléiques dont les extrémités 5′ sont détruites sur une longueur dépendant du temps d'action de la 5′ exonucléase, alors qu'elle ne détruit pas les extrémités 5′ ADN simple brin (amorces ou ADN dénaturé, par exemple).

Lors de l'étape (2) du procédé conforme à l'invention, les séquences d'acide nucléique courtes (séquences contaminantes) et les séquences d'acide nucléique longues (séquences comportant la séquence cible à détecter) vont, en raison de l'action du réactif de discrimination et plus particulièrement de la 5′ exonucléase, se comporter différemment :

- en ce qui concerne les séquences contaminantes, qui ne comprennent en fait que la séquence cible et les amorces d'amplification, incorporées aux extrémités, il n'y aura extension qu'à partir d'une seule amorce d'amplification, dans la mesure où les extrémités 5′ sont détruites ; une telle extension n'entraînera qu'une augmentation linéaire de ces séquences contaminantes ; si l'on considère que n cycles successifs d'amplification ont été effectués au cours de l'étape (2), les séquences contaminantes ne seront amplifiées que 2n fois, ce qui peut être considéré comme une amplification négligeable, par rapport à une amplification exponentielle qui entraîne la formation de $2^n$ séquences amplifiées.

- en ce qui concerne les séquences cibles à détecter, par contre, la destruction des extrémités 5′ n'affectera pas la zone de la séquence longue d'acide nucléique dans laquelle se trouvent les séquences cibles ; il y aura donc extension à partir des deux amorces d'amplification ; une telle extension entraînera une augmentation exponentielle desdites séquences cibles (production de $2^n$ séquences, lors de n cycles successifs d'amplification) et permettra une détection aisée desdites séquences.

La durée de l'action du réactif de discrimination et plus particulièrement de la 5′ exonucléase peut varier de quelques minutes à plus d'1/2 heure, selon les cas.

Le procédé conforme à l'invention permet donc, de manière inattendue, par la destruction des extrémités 5′ des acides nucléiques double-brin présents dans l'échantillon, d'éviter l'amplification exponentielle des séquences contaminantes, tout en n'affectant pas l'amplification exponentielle des séquences cibles incluses notamment dans un ADN génomique.

Il faut noter que, même dans le cas le moins favorable, c'est-à-dire lorsque la séquence cible se trouve à l'extrémité 5′ de l'ADN génomique, cette même séquence cible, sur le brin négatif, est loin de l'extrémité 5′ et sera donc à l'origine d'une amplification exponentielle.

L'étape (1) du procédé conforme à l'invention rend vulnérables les fragments d'acide nucléique double brin courts, alors que dans les fragments d'acides nucléiques longs, la séquence cible sera protégée et toujours amplifiée exponentiellement.

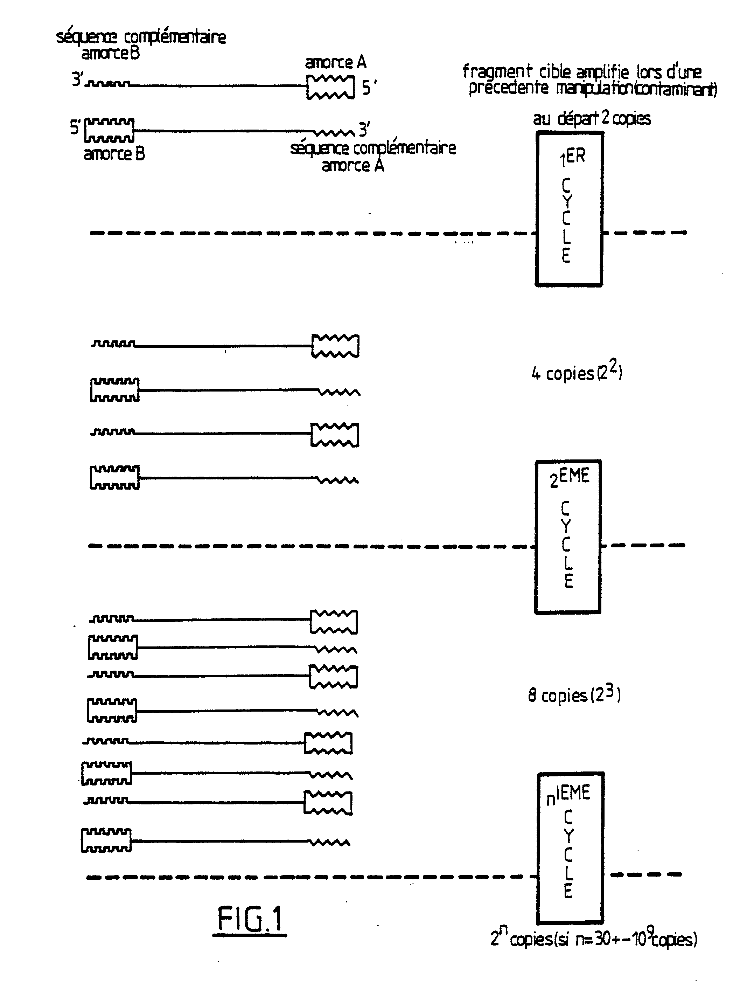

La figure 1 illustre le processus d'amplification exponentielle classique, obtenue par PCR, en partant d'un acide nucléique dit contaminant tel que défini ci-dessus, c'est-à-dire dont les extrémités sont définies par les extrémités 5' des amorces A et B utilisées.

Lors d'une telle amplification, il y aura extension des brins à partir des deux amorces et la séquence contaminante va être amplifiée exponentiellement, même en l'absence de séquences cibles dans l'échantillon à analyser : au bout de n cycles, on obtient $2^n$ exemplaires de la séquence contaminante. En 20 cycles, la séquence initiale est en principe amplifiée un million de fois.

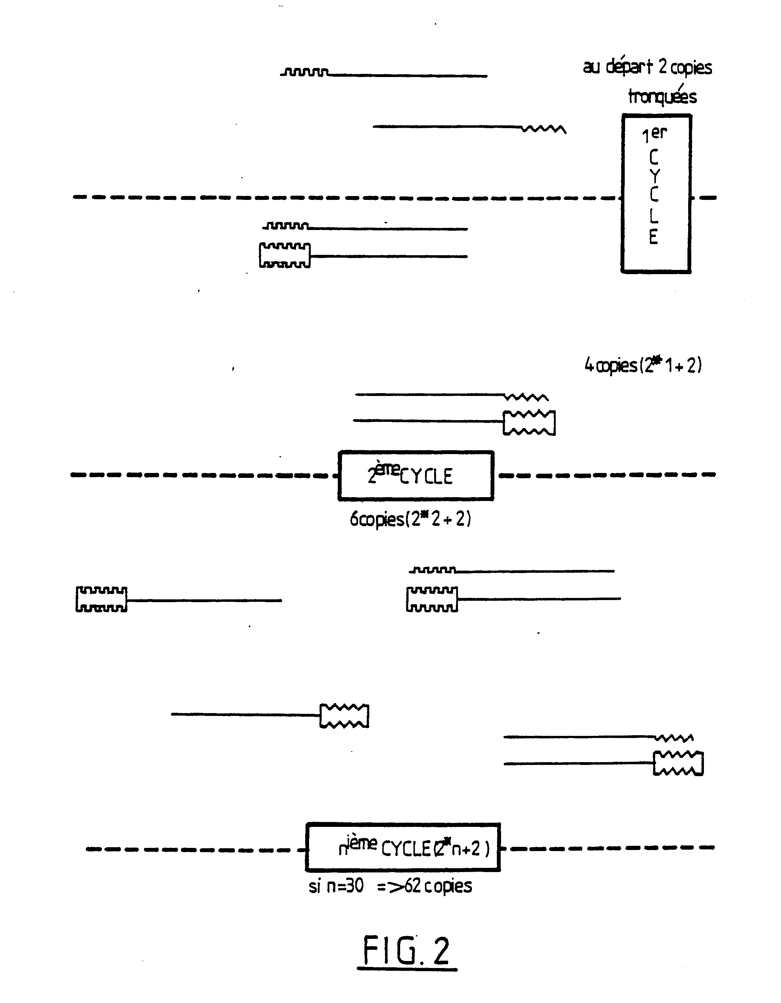

Par contre, lors de la mise en oeuvre du procédé conforme à l'invention, les séquences contaminantes, en présence de 5' exonucléase par exemple, ne conduisent pas à l'obtention de faux positifs, car elles ne sont amplifiées que linéairement, comme le montre la figure 2, où l'on voit qu'il n'y a extension qu'à partir d'une seule amorce et non des deux.

En effet, comme précisé ci-dessus, une amplification linéaire pour n cycles conduit seulement à la formation de 2n séquences.

Le Tableau I ci-après met en valeur le nombre de copies obtenues après amplification d'une séquence dite contaminante, sans traitement préalable avec une 5' exonucléase (colonne A et figure 1, amplification exponentielle classique), et avec traitement préalable avec une 5' exonucléase (colonne B et figure 2, amplification linéaire).

TABLEAU I

| Cycles | A Contaminant non traité | B Contaminant modifié |
|---|---|---|
| 1 | 2 | 2 |
| 2 | 4 | 3 |
| 3 | 8 | 4 |
| 4 | 16 | 5 |
| 5 | 32 | 6 |
| 6 | 64 | 7 |
| 7 | 128 | 8 |
| 8 | 256 | 9 |
| 9 | 512 | 10 |
| 10 | 1024 | 11 |
| 11 | 2048 | 12 |
| 12 | 4096 | 13 |
| 13 | 8192 | 14 |
| 14 | 16384 | 15 |
| 15 | 32768 | 16 |
| 16 | 65536 | 17 |
| 17 | 131072 | 18 |
| 18 | 262144 | 19 |
| 19 | 524288 | 20 |
| 20 | 1048576 | 21 |
| 21 | 2097152 | 22 |
| 22 | 4194304 | 23 |
| 23 | 8388608 | 24 |
| 24 | 16777216 | 25 |
| 25 | 33554432 | 26 |
| 26 | 67108864 | 27 |
| 27 | 134217728 | 28 |
| 28 | 268435456 | 29 |
| 29 | 536870912 | 30 |
| 30 | 1073741824 | 31 |

Selon un autre mode de mise en oeuvre avantageux dudit procédé, l'étape (1), à savoir l'étape de destruction des extrémités 5' des séquences d'acide nucléique double brin, n'est réalisée qu'une fois alors que l'étape (2) est répétée au moins une fois.

En effet, dans le procédé conforme à l'invention, le réactif de discrimination et plus particulièrement la 5' exonucléase ne doit agir qu'une fois, avant le premier cycle de l'étape d'amplification, pour éviter l'amplification exponentielle des séquences contaminantes ; il doit donc être thermosensible, pour pourvoir être aisément utilisable dans un procédé automatisé.

Selon un autre mode de mise en oeuvre avantageux de l'invention, l'étape (2) dudit procédé est réalisée, en outre, en présence d'une ADN polymérase thermosensible.

L'association d'une polymérase thermosensible, active à 37°C à la polymérase thermorésistante a l'avantage de permettre de résoudre au moins deux problèmes liés à la mise en oeuvre d'une PCR :
- de réparer les séquences contaminantes obtenues à partir d'une PCR, dont les derniers cycles d'amplifications ont de mauvais rendements, (obtention de contaminants simple brins, par exemple qui ne seraient donc pas détruits par le procédé conforme à l'invention),
- dans le cas de l'application de la PCR à de l'ARN, l'association d'une ADN polymérase ARN dépendante à la polymérase thermorésistante permet de réaliser la PCR sur des particules virales à ARN, en une seule étape.

Le procédé conforme à l'invention a l'avantage de modifier, en début de réaction, les fragments cibles amplifiés lors de précédentes manipulations (contaminants), sans modifications importantes de l'ADN plasmidique ou génomique dans lequel se trouve la séquence cible à détecter. Après ce traitement, le fragment cible amplifié préexistant ne peut plus servir de matrice pour une amplification exponentielle, alors que l'ADN plasmidique ou génomique reste toujours la matrice de départ.

La présente invention a également pour objet une composition enzymatique pour la mise en oeuvre d'un procédé de détection conforme à l'invention, caractérisée en ce qu'elle comprend un réactif détruisant les extrémités 5' des séquences d'acide nucléique double brin, dit réactif de discrimination entre séquences contaminantes et séquences cibles et une ADN polymérase thermorésistante.

Selon un mode de réalisation avantageux de ladite composition, ledit réactif de discrimination est une 5' exonucléase thermosensible.

Selon un autre mode de réalisation avantageux de ladite composition, elle comprend, en outre, une ADN polymérase active à 37°-42°C (thermosensible).

La présente invention a, en outre, pour objet un coffret, kit ou ensemble coordonné, prêt à l'emploi, pour la mise en oeuvre du procédé conforme à l'invention, caractérisé en ce qu'il comprend outre des quantités utiles de tampons et réactifs convenables, d'au moins une paire d'amorces appropriée et éventuellement d'au moins une sonde appropriée, des doses appropriées d'une composition enzymatique conforme à l'invention.

Outre les dispositions qui précèdent, l'invention comprend encore d'autres dispositions, qui ressortiront de la description qui va suivre, qui se réfère à des exemples de mise en oeuvre du procédé objet de la présente invention.

Il doit être bien entendu, toutefois, que ces exemples sont donnés uniquement à titre d'illustration de l'objet de l'invention, dont ils ne constituent en aucune manière une limitation.

De manière générale, le procédé conforme à l'invention permet d'éliminer les faux positifs dus à la contamination par des séquences identiques aux séquences à détecter, préalablement amplifiées lors de tests antérieurs, au laboratoire.

**Exemple 1 : Détection de _Chlamydia trachomatis_ par le procédé conforme à l'invention, en présence d'ADN polymérase thermorésistante ; comparaison avec une amplification classique (PCR).**

Des échantillons d'ADN de _Chlamydia_ obtenus à partir du plasmide cryptique de cette bactérie sont soumis à une amplification dans un milieu de réaction contenant en un volume total de 50 µl : 1 µM de chacune des amorces appropriées phosphorylées en 5', 200 µM de chacun des 4 désoxyribonucléotides triphosphates (dATP, dCTP, dGTP, dTTP), Tris 10 mM pH 8 ; KCl 50 mM ; MgCl₂ 5 mM, 1 unité de 5'3' polymérase thermorésistante et une unité de 5' lambda exonucléase thermosensible.

Le protocole est le suivant :
les échantillons sont chauffés à 37°C pendant 15 minutes (action de l'exonucléase) et sont ensuite soumis à une amplification comprenant 25 cycles, chaque cycle comportant : chauffage à 92°C pendant 15 secondes, refroidissement à 55°C pendant une minute puis chauffage à 72°C pendant une minute. Les échantillons sont alors conservés à 72°C pendant 10 minutes.

Les produits de la PCR sont séparés par électrophorèse sur gel d'agarose (1,4 %), préalablement coloré au bromure d'éthidium.

L'ADN est alors détecté par exposition aux rayons U.V..

En comparaison, on réalise des PCR classiques (sans ajout de 5′ exonucléase), selon le même protocole.

Cette comparaison a pour but de montrer les avantages du procédé conforme à l'invention, à savoir que la lambda exonucléase n'a pas d'effet sur le rendement des PCR faites à partir d'ADN génomique alors qu'elle diminue fortement le rendement de la PCR réalisée à partir de séquences contaminantes telles que définies ci-dessus, phosphorylées à leur extrémité 5′.

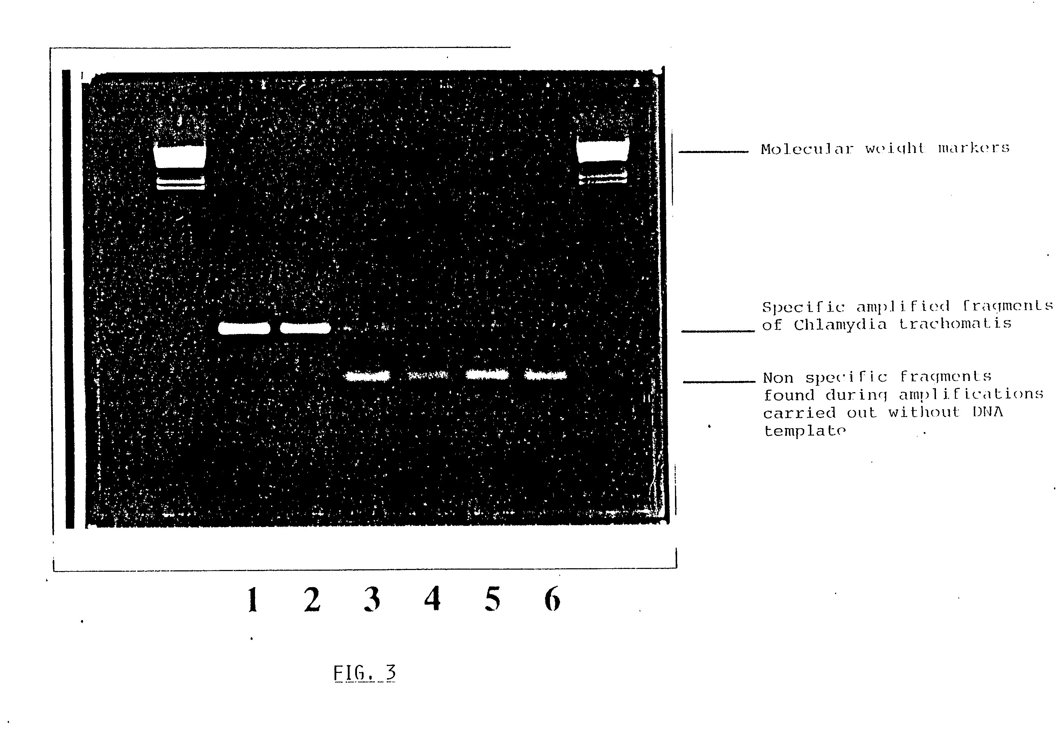

La figure 3, dans son ensemble, illustre cette comparaison et montre l'élimination des faux positifs, obtenue à l'aide du procédé conforme à l'invention, lorsque des produits de PCR réalisés dans les conditions différentes précisées ci-dessus, sont séparés par électrophorèse sur gel d'agarose (1,4 %), coloré au bromure d'éthidium, comme précisé ci-dessus.

Elle comprend :
- en colonne 1 : les produits d'amplification obtenus par PCR classique, en présence d'amorces phosphorylées, à partir d'un échantillon contenant un plasmide cryptique de *Chlamydia* ;
- en colonne 2 : les produits d'amplification obtenus par le procédé conforme à l'invention (action de la 5′ lambda exonucléase + PCR), en présence d'amorces phosphorylées, à partir d'un échantillon contenant un plasmide cryptique de *Chlamydia* ;
- en colonne 3 : les produits d'amplification obtenus par PCR classique, en présence d'amorces phosphorylées, à partir d'un échantillon négatif contenant des contaminants phosphorylés à leur extrémité 5′ ;
- en colonne 4 : les produits d'amplification obtenus par le procédé conforme à l'invention (action de la 5′ lambda exonucléase + PCR), en présence d'amorces phosphorylées, à partir d'un échantillon négatif contenant des contaminants phosphorylés à leur extrémité 5′ ;
- en colonne 5 : les produits d'amplification obtenus par une PCR classique, à partir d'un échantillon négatif contenant des contaminants non phosphorylés à leur extrémité 5′ ;
- en colonne 6 : les produits d'amplification obtenus par le procédé conforme à l'invention (5′ lambda exonucléase + PCR), à partir d'un échantillon négatif contenant des contaminants non phosphorylés à leur extrémité 5′.

Il ressort de cette figure que :
- la présence de 5′ lambda exonucléase n'influe pas sur l'amplification elle-même (colonne 1 et colonne 2) ;
- en l'absence de 5′ lambda exonucléase, un échantillon négatif donne un résultat faussement positif (colonne 3), alors que ce mauvais résultat disparaît, en présence de 5′ lambda exonucléase (colonne 4) ; il faut noter qu'en colonnes 3 et 4, on observe une légère bande qui correspond à des fragments non spécifiques, bande habituellement retrouvée lors d'amplifications conduites en l'absence d'une matrice d'ADN.
- lorsque l'on utilise de la 5′ lambda exonucléase, les contaminants phosphorylés à leur extrémité 5′ sont beaucoup plus sensibles à cette dernière (colonnes 5 et 6).

**Exemple 2 : Detection de *Chlamydia trachomatis* par le procédé conforme à l'invention.**

La réaction est mise en oeuvre comme suit :

Des échantillons d'ADN de *Chlamydia* issus d'échantillons cliniques sont ajoutés à un tampon de réaction contenant : Tris-HCl (pH 8,4) 10 mM ; KCl 50 mM ; MgCl₂ 2 mM ; désoxyribonucléotide triphosphates (dNTP) : 200 μM de chaque, amorce : 1 μM de chaque, 0,03 unité d'ADN lambda exonucléase par μl de réaction, 0,02 unité d'ADN polymérase thermorésistante par μl de réaction.

Les échantillons sont chauffés à 37°C pendant 20 minutes et sont soumis aux 25 cycles successifs suivants : les échantillons sont chauffés à 90°C pendant 15 secondes, refroidis à 55°C pendant une minute et chauffés à 72°C pendant une minute. Les échantillons sont alors conservés à 72°C pendant 10 minutes.

Les produits de la PCR obtenus avec les amorces suivantes :

amorce 1 (+) : 5′ - TTCCCCTTGTAATTCGTTGC - 3′

amorce 2 (-) : 5′ - TAGTAACTGCCACTTCATCA - 3′,

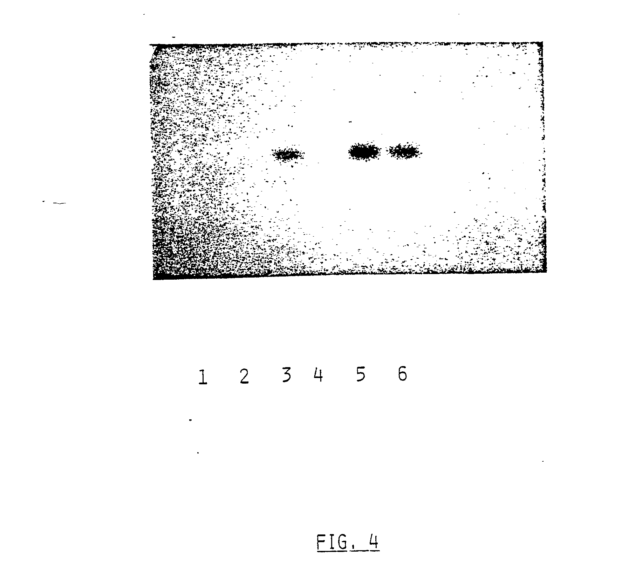

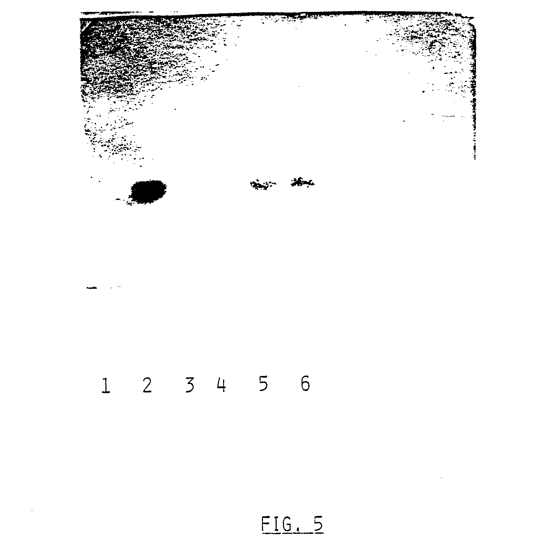

phosphorylées de manière classique par une polynucléotide kinase, présentent 201 paires de bases et sont séparés sur gel d'agarose coloré au bromure d'éthidium. L'ADN est alors détecté par exposition aux rayons U.V.. Les produits de la PCR peuvent également être révélés par autoradiographie, après la mise en oeuvre d'un Southern Blot sur filtre Hybond N (Amersham) avec une sonde d'ARN simple brin, spécifique desdits produits, marquée soit au $^{32}$P (figure 4 : colonne 1 : marqueurs ; colonne 2 : témoin négatif ; colonne 3 : témoin positif ; colonne 4 : échantillon clinique négatif ; colonnes 5 et 6 : échantillons cliniques positifs) soit au Dig-dUTP (figure 5 : colonne 1 : marqueurs ; colonne 2 : témoin positif ; colonne 3 : témoin négatif ; colonne 4 : échantillon clinique négatif ; colonnes 5 et 6 : échantillons cliniques positifs). Les échantillons cliniques contenant de l'ADN génomique de *Chlamydia trachomatis* sont bien détectés en présence de lambda exonucléase (figure 4 : colonnes 3 et 4 ; figure 5 : colonnes 5 et 6).

**Exemple 3 : Rôle de la concentration en contaminants.**

On prépare des séquences dites contaminantes de cytomégalovirus (CMV).

Il s'agit de produits d'extensions d'amorces phosphorylées, obtenues lors d'amplifications antérieures. Ces séquences contaminantes contiennent donc uniquement des séquences cibles contenant à leurs extrémités lesdites amorces phosphorylées.

Lesdites séquences contaminantes sont obtenues par purification à partir d'une électrophorèse sur gel d'agarose, puis par électroélution, précipitation à l'éthanol, mise en suspension dans de l'eau et quantification avec "DNA DIPSTRICK from INVITROGEN 1-800-544-4684".

Les séquences contaminantes ainsi obtenues sont ensuite diluées, de manière à obtenir environ $10^5$ copies dans 15 $\mu$l d'eau. Des dilutions en série, d'ordre 5, sont ensuite effectuées, pour pouvoir évaluer le nombre de copies présent dans cha ... e échantillon, avant de réaliser chaque PCR.

Les différents échantillons sont soumis soit à une PCR classique (absence de 5' lambda exonucléase), soit à une PCR en présence de 5' lambda exonucléase (procédé conforme à l'invention).

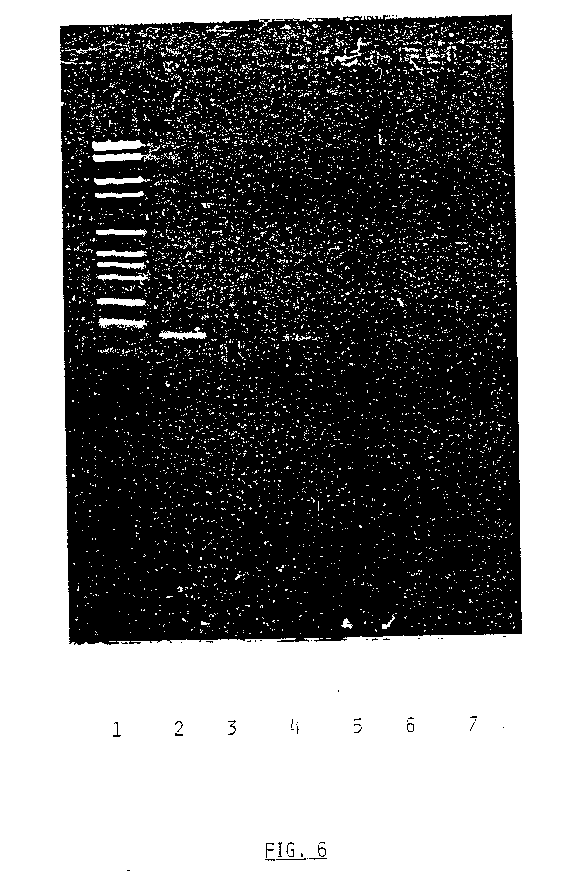

Le rôle de la 5' lambda exonucléase, en fonction de la concentration en produit contaminant, est montré sur la figure 6.

Les produits de la PCR sont séparés par électrophorèse sur gel d'agarose coloré au bromure d'éthidium et l'ADN est détecté par exposition aux rayons U.V.

La figure 6 montre les résultats suivants :
- en colonne 1, on a des marqueurs de poids moléculaire (Marker VI, Boehringer) ;
- en colonne 2 : $10^5$ copies de séquences CMV contaminantes amplifiées en l'absence de 5' lambda exonucléase ;
- en colonne 3 : $10^5$ copies de séquences CMV contaminantes amplifiées, après 20 minutes de contact avec de la lambda exonucléase (0,03 U/$\mu$l) ;
- en colonne 4 : $2.10^4$ copies de séquences CMV contaminantes amplifiées en l'absence de lambda exonucléase ;
- en colonne 5 : $2.10^4$ copies de séquences CMV contaminantes amplifiées après 20 minutes de mise en contact avec de la lambda exonucléase (0,03 U/$\mu$l) ;
- en colonne 6 : $4.10^3$ copies de séquences CMV contaminantes amplifiées en l'absence de lambda exonucléase ;
- en colonne 7 : $4.10^3$ copies de séquences CMV contaminantes amplifiées en présence de lambda exonucléase (0,03 U/$\mu$l, temps de contact : 20 minutes).

Il ressort de cette figure :
- qu'en l'absence de lambda exonucléase, on obtient une bande correspondant aux séquences CMV (colonnes 2, 4 et 6) ;
- alors qu'en présence de lambda exonucléase, et quelle que soit la concentration en séquences contaminantes, on n'obtient aucune bande (colonnes 3, 5 et 7).

**Exemple 4 : Rôle de la lambda exonucléase sur l'ADN génomique contenant une séquence cible à détecter.**

Différents ADN génomiques ont été utilisés, pour évaluer le rôle de la lambda exonucléase sur différents ADN. Différents herpès virus et le virus du papillome ont été plus particulièrement étudiés.

1) Herpès virus :

Les ADN sont obtenus à partir de lignées cellulaires infectées par les herpès virus humains suivants : HSV2, CMV, VZ et EBV.

Des dilutions en série sont réalisées avant d'effectuer les PCR en présence d'amorces appropriées auxdits différents virus, présentant de 20 à 30 nucléotides et phosphorylées de manière classique par une polynucléotide kinase.

Pour réaliser la PCR, lesdits échantillons sont ajoutés à un tampon contenant : Tris-HCl (pH 8,4) 10 mM ; KCl 50 mM ; MgCl₂ 2 mM ; gélatine ; 0,01 % dNTP : 200 µM de chaque ; amorces appropriées : 1 µM de chaque ; TAQ polymérase (CETUS PERBIN-ELMER) : 0,02 U/µl de réaction ; lambda exonucléase (BRL) O ou 0,03 U/µl de réaction selon la procédé mis en oeuvre (PCR classique ou procédé conforme à l'invention, respectivement).

Les échantillons sont soumis aux différents cycles thermiques suivants :
- 1 fois : 37° C pendant 20 minutes ; 92° C pendant 1 minute (action de la lambda exonucléase s'il y a lieu) ;
- 30 fois : 92° C pendant 15 secondes ; 55° C pendant 1 minute ; 72° C pendant 1 minute (PCR proprement dite) ;
- 1 fois : 72° C pendant 10 minutes.

Les produits d'amplification sont soumis à une électrophorèse en gel d'agarose 2 % coloré au bromure d'éthidium et photographiés sur plaques U.V..

Les marqueurs de poids moléculaire utilisés (Marker VI, Boehringer) ont les tailles suivantes : 2176, 1766, 1230, 1033, 653, 517, 453, 394, 298, 234, 220, 154.

Selon le virus détecté, on obtient les résultats suivants :

1.a. Virus Varicelle zona (VZV) :

Des dilutions sériées d'ordre 2 d'ADN génomique de cellules infectées par le virus varicelle zona, sont soumises à une PCR avec des amorces VZV phosphorylées appropriées, en présence et en l'absence de lambda exonucléase, comme précisé ci-dessus.

Les produits de la PCR obtenus avec les amorces suivantes :

amorce 1 (+) : 5′ - ATGGGGTATGCATACGTCGAGGCGGTTGAC - 3′
amorce 2 (-) : 5′ - ATATTGAAGCAAATCGCGACATCGTACTAC - 3′

présentent 189 paires de bases.

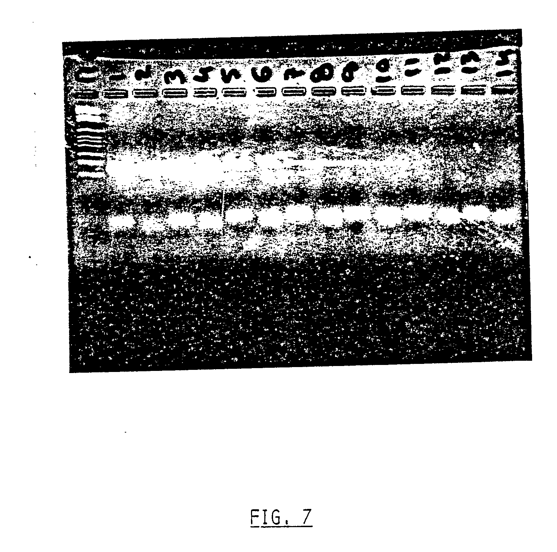

Après électrophorèse sur gel d'agarose, comme précisé ci-dessus, on obtient les résultats illustrés à la figure 7, dans laquelle les colonnes impaires représentant une PCR réalisée en l'absence de lambda exonucléase et les colonnes paires représentent une PCR réalisée en présence de lambda exonucléase.

Il ressort de cette figure, qu'une détection réalisée à partir d'un ADN génomique long n'est pas influencée par la présence de lambda exonucléase.

Les mêmes résultats sont obtenus sur d'autres herpès virus, comme précisé ci-dessus :

1.b. EBV :

Des dilutions sériées d'ordre 10 d'ADN génomique de cellules infectées par le virus d'Epstein-Barr, sont soumises à une PCR avec des amorces EBV phosphorylées appropriées, en présence et en l'absence de lambda exonucléase, comme précisé ci-dessus.

Les produits de la PCR obtenus avec les amorces suivantes :

amorce 1 (+) : 5′ - AGAGGTTCAGGGACTTGTCCTCTATCGTCT - 3′
amorce 2 (-) : 5′ - TTACCAACAACATGCTGATTCGCGACAACA - 3′

présentent 282 paires de bases.

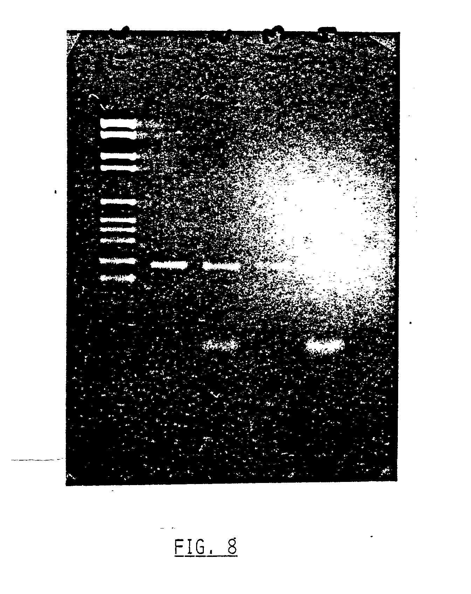

Après électrophorèse sur gel d'agarose, comme précisé ci-dessus, on obtient les résultats illustrés à la figure 8, dans laquelle les colonnes impaires représentent une PCR réalisée en l'absence de lambda exonucléase et les colonnes paires représentent une PCR réalisée en présence de lambda exonucléase.

1.c. HSV2 :

Des dilutions sériées d'ordre 10 d'ADN génomique de cellules infectées par le virus Herpès Simplex 2. sont soumises à une PCR avec des amorces HSV phosphorylées appropriées, en présence et en l'absence de lambda exonucléase.

Les produits de la PCR obtenus avec les amorces suivantes :
amorce 1 (+) : 5' - TTCCCGGTCGCCGGGCACCACCACGCCGTA - 3'
amorce 2 (-) : 5' - TTCGCTTCATCGCCACGAAGACCAACGACG - 3'
présentent 185 paires de bases.

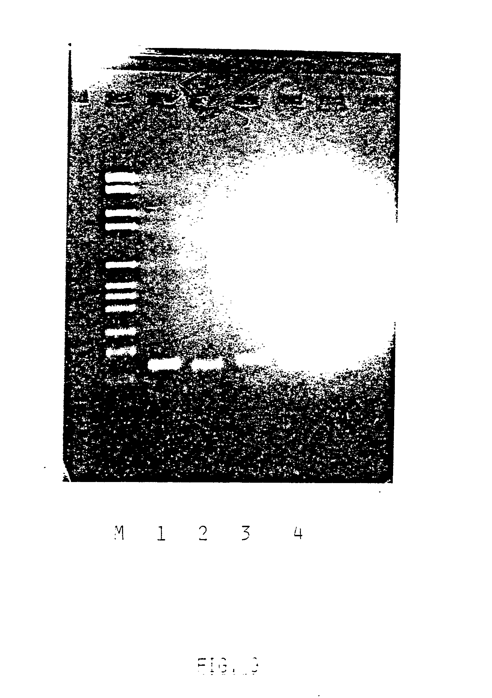

Après électrophorèse sur gel d'agarose, comme précisé ci-dessus, on obtient les résultats illustrés à la figure 9, dans laquelle les colonnes impaires représentent une PCR réalisée en l'absence de lambda exonucléase et les colonnes paires représentent une PCR réalisée en présence de lambda exonucléase.

Pour la détection du cytomégalovirus, on procède comme ci-dessus, en présence des amorces suivantes :
amorce 1 (+) : 5' - ACGGCGTTCCCCCATAAAGTCACGTAACAC - 3'
amorce 2 (-) : 5' - CGCCGAGAAACACGGCGGACGCATCGACGG - 3'
et l'on obtient un fragment amplifié de 259 paires de bases.

2) Virus du papillome (HPV 11) :

Des dilutions sériées d'ordre 2 d'ADN génomique, obtenu à partir d'un condylome anal prélevé sur un patient, sont soumises à une PCR selon le protocole décrit ci-dessus en 1), avec des amorces HPV 11 phosphorylées appropriées, en présence et en l'absence de lambda exonucléase.

Les produits de la PCR obtenus avec les amorces suivantes :
amorce 1 (+) : 5' - TGGTACTTTGCACGCAGACGCCGTA - 3'
amorce 2 (-) : 5' - ATGATAAAATATGTTGGTGCGTTTA - 3'
présentent 154 paires de bases.

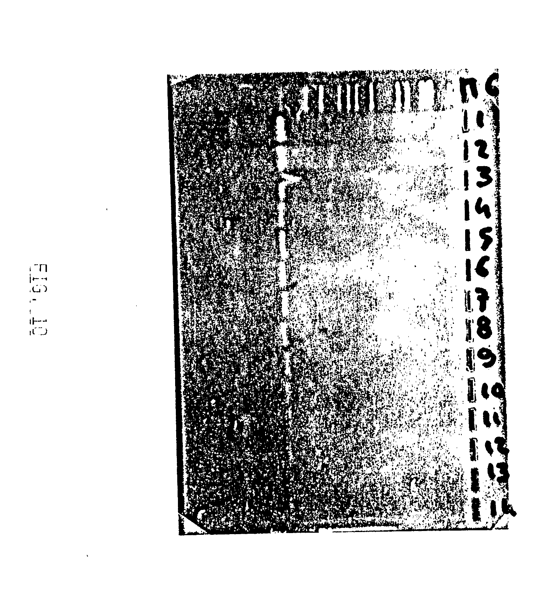

Après électrophorèse sur gel d'agarose, comme précisé ci-dessus, on obtient les résultats illustrés à la figure 10, dans laquelle les colonnes impaires représentent une PCR réalisée en l'absence de lambda exonucléase et les colonnes paires représentent une PCR réalisée en présence de lambda exonucléase.

Les résultats illustrés sur les figures 7 à 10 montrent que la lambda exonucléase ne modifie pas le rendement d'une amplification réalisée à partir d'une matrice d'ADN génomique (fragment long).

## Exemple 5 : Rôle de la lambda exonucléase sur des séquences dites contaminantes.

Les ADN contaminants sont isolés par électrophorèse sur gel d'agarose puis sont ensuite soumis à une diffusion passive à 4° C dans de l'eau.

Les produits contaminants ainsi obtenus sont dilués comme précisé ci-dessus à l'exemple 4.1) et le protocole PCR est identique à celui décrit à l'exemple 4.1).

Les résultats obtenus avec CMV, EBV et VZV comme séquences contaminantes sont illustrés aux figures 11, 12 et 13 et montrent que la présence d'exonucléase diminue très fortement le rendement d'amplification de ces séquences contaminantes.

a. CMV :

Les dilutions sériées d'ordre 5 de produits d'amplification obtenus par PCR avec des amorces CMV phosphorylées appropriées, sont soumises à une PCR avec les mêmes amorces, en présence et en l'absence de lambda exonucléase.

Après électrophorèse sur gel d'agarose, comme précisé ci-dessus à l'exemple 4, on obtient les résultats illustrés à la figure 11, dans laquelle les colonnes impaires représentent une PCR réalisée en l'absence de lambda exonucléase et les colonnes paires représentent une PCR réalisée en présence de lambda exonucléase.

b. EBV :

Des dilutions sériées d'ordre 5 de produits d'amplification obtenus par PCR avec des amorces EBV phosphorylées appropriées, sont soumises à une PCR avec ces mêmes amorces, en présence et en l'absence de lambda exonucléase.

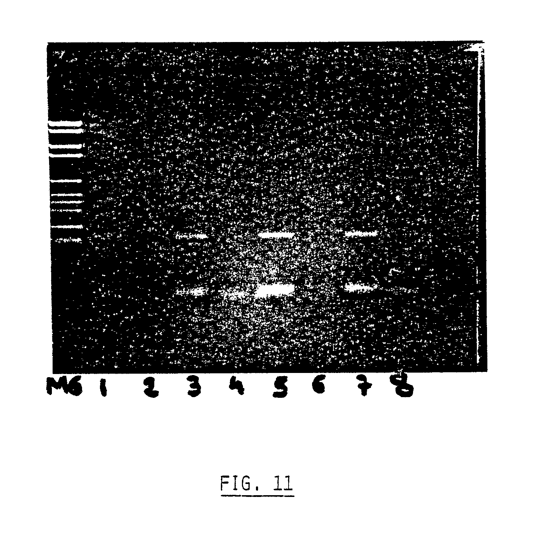

Après électrophorèse sur gel d'agarose, comme précisé ci-dessus, on obtient les résultats illustrés à la figure 12, dans laquelle les colonnes impaires représentent une PCR réalisée en l'absence de lambda exonucléase et les colonnes paires représentent une PCR réalisée en présence de lambda exonucléase.

c. VZV :

Des dilutions sériées d'ordre 5 de produits d'amplification obtenus par PCR avec des amorces VZV phosphorylées appropriées, sont soumises à une PCR avec ces mêmes amorces, en présence et en l'absence de lambda exonucléase.

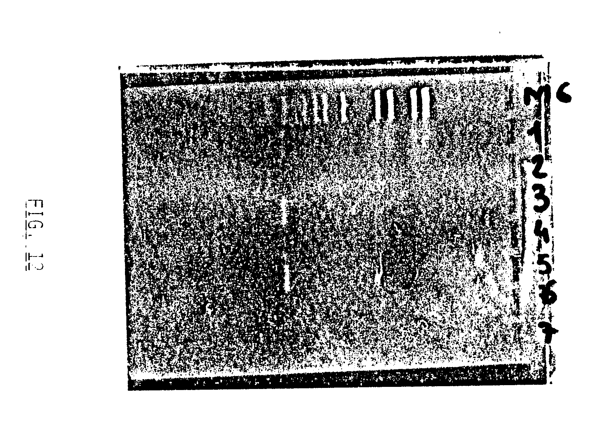

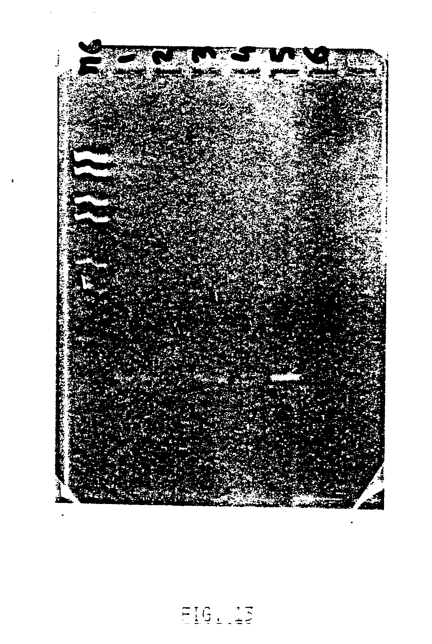

Après électrophorèse sur gel d'agarose, comme précisé ci-dessus, on obtient les résultats illustrés à la figure 13, dans laquelle les colonnes impaires représentent une PCR réalisée en l'absence de lambda exonucléase et les colonnes paires représentent une PCR réalisée en présence de lambda exonucléase.

**Exemple 6 : Action de concentrations croissantes de lambda exonucléase sur le rendement d'amplification d'ADN génomique.**

Une quantité constante d'ADN génomique obtenue à partir de cellules infectées par VZV est soumise à une PCR en présence de quantités croissantes de lambda exonucléase (0 ; 0,36 ; 0,18 ; 0,09 ; 0,045 ; 0,0225 U/$\mu$l) suivant le protocole précédemment décrit, à l'exemple 4 ci-dessus.

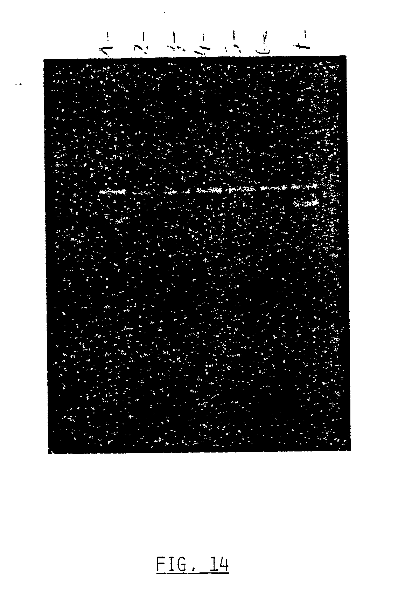

Les résultats sont illustrés à la figure 14, dans laquelle les colonnes 1 à 7 correspondent successivement aux différentes concentrations de lambda exonucléase précisées ci-dessus. Cette figure montre que 0,09 U/$\mu$l de lambda exonucléase ne diminue pas le rendement de la PCR ; ceci montre qu'une concentration de 0,03 U/$\mu$l de réaction est tout à fait convenable.

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée, de la présente invention.

**Revendications**

1°) Procédé de détection et/ou d'identification d'une séquence d'acide nucléique ou d'un mélange de séquences d'acides nucléiques comprenant une amplification enzymatique, caractérisé en ce qu'après mise en solution appropriée de l'échantillon biologique, pour extraire le/les acides nucléiques, ledit procédé comprend les étapes suivantes :
   (1) une étape de destruction des extrémités 5' des séquences d'acide nucléique double brin présentes dans l'échantillon, par mise en contact dudit échantillon biologique avec un réactif approprié, actif à une température comprise entre 37°-42°C ;
   (2) une étape d'amplification proprement dite par mise en contact de l'échantillon obtenu en (1) avec des réactifs appropriés, notamment des amorces d'amplification appropriées à l'amplification de la/les séquences cibles à détecter, en présence d'une ADN polymérase thermorésistante ;
   (3) une étape de détection des séquences cibles spécifiques amplifiées.
2°) Procédé selon la revendication 1, caractérisé en ce que le réactif de l'étape (1), dénommé réactif de discrimination entre séquences contaminantes et séquences cibles, est choisi dans le groupe qui comprend des produits chimiques convenables et des enzymes qui agissent en milieu alcalin faible.
3°) Procédé selon la revendication 2, caractérisé en ce que l'enzyme est une 5' exonucléase.
4°) Procédé selon la revendication 3, caractérisé en ce que la 5' exonucléase est la 5' lambda exonucléase.
5°) Procédé selon la revendication 4, caractérisé en ce que les amorces mises en oeuvre pour l'amplification de l'étape (2) sont phosphorylées à leurs extrémités 5'.
6°) Procédé selon l'une quelconque des revendications 1 à 5, caractérisé en ce que l'étape (1), à savoir l'étape de destruction des extrémités 5' des séquences d'acide nucléique double brin, n'est réalisée qu'une fois alors que l'étape (2) est répétée au moins une fois.
7°) Procédé selon l'une quelconque des revendications 1 à 6, caractérisé en ce que l'étape (2) dudit procédé est réalisée, en 'outre, en présence d'une ADN polymérase thermosensible.
8°) Composition enzymatique pour la mise en oeuvre d'un procédé de détection selon l'une quelconque

11

des revendications 1 à 7, caractérisée en ce qu'elle comprend un réactif détruisant les extrémités 5' des séquences d'acide nucléique double brin, dit réactif de discrimination entre séquences contaminantes et séquences cibles et une ADN polymérase thermorésistante.

9°) Composition selon la revendication 8, caractérisée en ce que ledit réactif de discrimination est une 5' exonucléase thermosensible.

10°) Composition selon l'une quelconque des revendications 8 et 9, caractérisée en ce qu'elle comprend, en outre, une ADN polymérase active à 37°-42° C (thermosensible).

11°) Coffret, kit ou ensemble coordonné, prêt à l'emploi, pour la mise en oeuvre du procédé selon l'une quelconque des revendications 1 à 7, caractérisé en ce qu'il comprend outre des quantités utiles de tampons et réactifs convenables, d'au moins une paire d'amorces appropriée et éventuellement d'au moins une sonde appropriée, des doses appropriées d'une composition enzymatique selon l'une quelconque des revendications 8 à 10.

séquence complémentaire
amorce B

3′ ⌐⌐⌐⌐⌐ _____ ⌐⌐⌐⌐ 5′     amorce A

5′ ⌐⌐⌐⌐⌐ _____ ⌐⌐⌐⌐ 3′
amorce B                    séquence complémentaire
                            amorce A

fragment cible amplifie lors d'une
precedente manipulation (contaminant)

au départ 2 copies

1ER CYCLE

4 copies $(2^2)$

2EME CYCLE

8 copies $(2^3)$

nIEME CYCLE

FIG.1

$2^n$ copies (si n=30 +− $10^9$ copies)

au départ 2 copies
tronquées

1er CYCLE

4 copies (2*1 + 2)

2ème CYCLE

6 copies (2*2 + 2)

nième CYCLE (2*n + 2)

si n=30  => 62 copies

FIG. 2

Molecular weight markers

Specific amplified fragments of Chlamydia trachomatis

Non specific fragments found during amplifications carried out without DNA template

1 2 3 4 5 6

FIG. 3

1  2  3  4  5  6

FIG. 4

1   2   3   4   5   6

FIG. 5

FIG. 6

FIG. 7

FIG. 8

M  1  2  3  4

FIG. 9

FIG. 10

FIG. 11

FIG. 12

EP 0 407 291 A1

FIG. 13

FIG. 14

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| P,A | WO-A-8 909 285  (BOARD OF REGENTS, THE UNIVERSITY OF TEXAS SYSTEM)(10-1989) * Voir le document en entier, particulièrement page 8, lignes 3-6 * --- | 1-4,7-10 | C 12 Q   1/68 C 12 Q   1/48  // C 12 Q   1/70 C 07 H  21/04 |
| A | NUCLEIC ACIDS RESEARCH, vol. 17, no. 14, 25 juillet 1989, page 5865, IRL Press; R.G. HIGUCHI et al.: "Production of single-stranded DNA templates by exonuclease digestion following the polymerase chain reaction" * Voir l'article en entier * --- | 1-4 | |
| A | EP-A-0 237 362  (CETUS CORP.)(06-1986) * Voir le document en entier, particulièrement page 16, lignes 18-21 * --- | 1-4,7-10 | |
| A | WO-A-8 607 612   (AMERSHAM INTERNATIONAL PLC)(12-1986) --- | | |
| A | EP-A-0 227 976  (MEIOGENICS INC.)(07-1987) ----- | | |

|  | DOMAINES TECHNIQUES RECHERCHES (Int. Cl.5) |
|---|---|
|  | C 12 Q |

Le présent rapport a été établi pour toutes les revendications

| Lieu de la recherche | Date d'achèvement de la recherche | Examinateur |
|---|---|---|
| LA HAYE | 11-10-1990 | OSBORNE H.H. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)